# DEMANDE DE BREVET EUROPEEN

(11) **EP 1 044 686 A1**
(43) Date de publication de la demande: **18.10.2000**
(21) Numéro de dépôt: 00460026.8
(22) Date de dépôt: 05.04.2000
(51) Int. Cl.: A61K 31/155, A61K 47/10, A61K 9/08

(54) **Solution de chlorhexidine alcoolique colorée et sa méthode de fabrication**

(30) Priorité: 12.04.1999 FR 9904572
(71) Demandeur: Laboratoires Gilbert SA, 14204 Herouville-Saint Clair Cedex (FR)
(72) Inventeur: Dronne, Jean-Jacques, 14920 Mathieu (FR)
(74) Mandataire: Maillet, Alain

(57) **Abrégé**

La solution est destinée à une application cutanée et, particulièrement, a l'antisepsie du champ opératoire qui est préparé en vue d'une intervention chirurgicale.

Elle comprend une principe actif, composé de digluconate de chlorhexidine et d'éthanol, et un produit colorant, composé d'un mélange de deux produits, qui fait que la solution appliquée sur la peau est de coloration verte.

Une méthode de fabrication de la solution comprend les étapes successives suivantes :
- on commence par préparer les colorants, c'est-à-dire qu'on mélange 142 ml d'eau déminéralisée, 250 mg de jaune de quinoléine et 50 mg de bleu patenté, puis qu'on agite le mélange pendant 10 minutes ;
- on ajoute au mélange 422 ml d'alcool éthylique à 95 % et 15 g de chlorhexidine ; on agite l'ensemble pendant 10 minutes ;
- on procède à une filtration clarifiante en utilisant un filtre 0,22 µm.

## Description

La présente invention concerne une solution de chlorhexidine alcoolique colorée destinée à une application cutanée et, plus particulièrement, à l'antisepsie du champ opératoire qui est préparé en vue d'une intervention chirurgicale. L'invention concerne également une méthode de fabrication de ladite solution de chlorhexidine alcoolique colorée.

On sait que la chlorhexidine possède une activité désinfectante marquée qui est décrite dans les documents US-A-3 637 767 et US-A-5 308 611 qui mettent en évidence les propriétés bactéricides de la chlorhexidine que l'on peut utiliser en médecine et en chirurgie pour la stérilisation d'instruments et de tissus, ou dans des préparations de cosmétiques.

Dans le document FR-A-2 516 919, on a proposé l'utilisation de dinaproxénate de chlorhexidine pour son activité anti-inflammatoire, désinfectante et anti-bactérienne, et qui présente une bio-disponibilité élevée par voie cutanée, ce qui permet un emploi efficace du composé par voie topique. A titre d'exemple, le composé est proposé dans des crèmes et des lotions dermatologiques, des collutoires, des suspensions à usage gynécologique et des poudres sèches. On doit noter toutefois qu'il est difficilement soluble dans l'eau et dans les solvants organiques courants, mais qu'il se dissout dans le diméthyl-sulfoxyde, même à froid.

Dans le document US-A-5 308 611, il est décrit des compositions antiseptiques, contenant de la chlorhexidine et un composé tensio-actif, qui moussent peu et peuvent être utilisées dans la désinfection d'hôpitaux, de cliniques et de laboratoires.

Dans le document WO-A-93/07250, il est décrit des compositions qui sont destinées à nettoyer particulièrement les mains et qui comprennent un alcool, un concentré aqueux de tensio-actif et, en supplément, un bactériocide tel que du gluconate de chlorhexidine. On applique le produit sur la peau, on laisse l'alcool s'évaporer, on applique un peu d'eau et on lave, puis on rince la peau.

Dans le document DE-A-4 137 548, il est décrit des compositions antimicrobiennes qui comprennent de l'acriflavine, de la chlorhexidine, de l'iodophore et, éventuellement, une partie alcool, la combinaison des constituants créant une synergie qui améliore l'action antimicrobienne. L'acriflavine est connue comme antiseptique buccal coloré. On utilise la combinaison comme antiseptique et désinfectant de la peau, des muqueuses et des plaies.

On connaît aussi le document EP-A-0 300 961 qui concerne une solution anti-bactérie pour la bouche et qui comprend de la chorhexidine et une solution à base d'alcool, avec pour supplément l'addition d'un colorant de couleur verte à base de menthe pour être plus agréable dans la bouche. Cette solution ne peut être utilisée pour rendre un champ opératoire antiseptique car elle ne désinfecte qu'à un instant limité et précis et non longtemps comme on s'y attend pour un champ opératoire car elle contient un colorant destiné à être ingéré par la bouche.

On connaît aussi le document FR-A-96 10672 qui concerne une composition contenant du digluconate de chlorhexidine, du chlorure de myristalkodium et un polymère d'acide acrylique, avec encore, pour la même raison que précédemment, un colorant du genre vert menthe au goût agréable. La composition décrite dans ce document est destinée à la chirurgie dentaire, ce qui diffère d'une application sur la peau pour une longue durée.

Un objet de l'invention consiste à prévoir une solution à base de chlorhexidine alcoolique destinée à être appliquée en solution sur la peau, et plus particulièrement destinée à délimiter un champ opératoire pour une période de temps relativement longue, c'est-à-dire à désinfecter un champ opératoire. Il est évident que cette solution doit être obligatoirement colorée pour parfaitement délimiter la zone aseptisée, mais il n'y a pas de couleur imposée, bien qu'on préfère éviter le rouge.

Actuellement, on utilise, à cet effet, généralement deux produits : l'hibitane champ qui est une solution de chlorhexidine à 0,5 % dans de l'éthanol à 70° et dont la couleur est rouge ; et la bétadine, un composé iodé en milieu alcoolique, de couleur jaune brun, à cause du composé iodé.

Un autre objet de l'invention consiste à prévoir une solution qui présente des avantages qui sont absents dans les produits connus.

Suivant une caractéristique de l'invention, la solution comprend un principe actif, composé de digluconate de chlorhexidine et d'éthanol, et un produit colorant, composé d'un mélange de deux produits: jaune de quinoléine et bleu patenté, ce qui donne à la solution appliquée sur la peau une coloration verte.

Suivant une autre caractéristique de l'invention, la solution comprend les constituants suivants pour 100 ml:
Solution de digluconate de chlorhexidine à 20 % : 2,66 g
(ce qui correspond à 0,5 g de digluconate de chlorhexidine)
Ethanol à 95 % : 75,0 ml
Jaune de quinoléine : 44,4 mg
Bleu patenté : 8,9 mg
Eau purifiée : 25,2 ml

Par rapport à l'hibitane champ, la solution suivant l'invention, telle que définie ci-dessus, présente l'avantage de pouvoir être directement prête à l'emploi. Pour procéder à la préparation de l'antisepsie d'un champ opératoire en vue d'une opération chirurgicale avec de l'hibitane champ, le personnel doit se livrer obligatoirement à une manipulation préalable, car le colorant de ce produit, qui est livré en petite dose, doit être mélangé extemporanément. Cette manipulation obligatoire, avant utilisation de l'hibitane champ, peut s'avérer dangereuse pour le patient. En effet, dans le cadre de la prévention des infections nosocomiales, l'utilisation à l'hôpital de produits antiseptiques prêts à l'emploi, qui sont fabriqués industriellement selon les bonnes pratiques de fabrication pharmaceutiques, est recommandée comme facteur supplémentaire de prévention. C'est le cas de la solution suivant l'invention, qui a été définie ci-dessus.

Suivant une autre caractéristique, la méthode de fabrication de ladite solution, comprend les phases successives suivantes, la quantité unité de solution correspondant à 500 g :
- on commence par la préparation des colorants, c'est-à-dire qu'on mélange 142 ml d'eau déminéralisée, 250 mg de jaune de quinoléine et 50 mg de bleu patenté, puis on agite le mélange pendant 10 minutes ;
- on ajoute au mélange 422 ml d'alcool éthylique à 95 % et 15 g de chlorhexidine ; on agite l'ensemble pendant 10 minutes ;
- on procède à une filtration clarifiante.

Suivant une autre caractéristique, pour la filtration clarifiante, on utilise un filtre 0,22 µm.

Suivant une autre caractéristique, on conserve ladite solution dans des flacons opaques en polyéthylène haute densité.

On va maintenant décrire l'invention en commentant le choix de la formulation après avoir défini, d'une part, les constituants du principe actif, c'est-à-dire le digluconate de chlorhexidine et l'éthanol, et, d'autre part, les colorants.

L'éthanol, ici l'alcool éthylique à 95 %, est décrit dans la monographie «Alcool» de la Pharmacopée Française d'octobre 1991. C'est une substance stable, bien connue et couramment utilisée dans les formulations pharmaceutiques.

Le digluconate de chlorhexidine est décrit dans la monographie « Solution de digluconate de chlorhexidine » (1007, 658) de la Pharmacopée Européenne, troisième édition. La solution est stable pendant 4 ans à température ambiante. Le digluconate de chlorhexidine est un antiseptique bactéricide à large spectre, appartenant à la famille des bis-diguanides. On sait que, sous forme de base, la chlorhexidine est pratiquement insoluble dans l'eau. Par contre, le sel digluconate présente une bonne solubilité dans l'eau et les alcools. Il est très approprié aux solutions antiseptiques. Il faut encore savoir que le gluconate de chlorhexidine se dégrade partiellement au cours du temps, particulièrement après 6 mois de conservation à des températures excédant les 25° C. En se dégradant, il libère du p-chloroaniline dont le taux peut s'élever jusqu'à 1,9 %.

Au point de vue chimique, la chlorhexidine se comporte comme un antiseptique cationique. Elle est bactériostatique et bactéricide sur un large spectre de bactéries Gram positif (propriété de bactéries de retenir la couleur quand elle ont été teintes par certaines couleurs à l'aniline et des iodures, et traitées par un agent décolorant comme l'alcool) et Gram négatif (propriété de bactéries d'être décolorées par un agent décolorant, quand elles ont été teintes par certaines couleurs à l'aniline ou des iodures). Elle possède encore une activité sur un certain nombre de champignons et de moisissures.

L'éthanol ou alcool éthylique, à la concentration optimale de 95 % (v/v), renforce le pouvoir bactéricide.

Le principe actif seul, c'est-à-dire comprenant uniquement éthanol et gluconate de chlorhexidine, est incolore, ce qui ne convient pas à la délimitation du champ opératoire, il faut donc ajouter un produit colorant.

En ce qui concerne le choix du produit colorant composé d'un colorant ou d'une combinaison de plusieurs colorants, on a défini un certain nombre de conditions : le produit colorant doit être directement intégré dans la formulation ; on a choisi de lui donner une coloration finale différente de la couleur rouge ; cette coloration doit être stable au cours du temps, sans interaction avec le principe actif; il doit répondre à une monographie de la Pharmacopée Française ou Européenne.

On a d'abord essayé le jaune de quinoléine de coloration jaune et l'érythrosine de coloration rouge et on a constaté que cette dernière, si elle donne une coloration vive sur la peau pour une concentration de 50 mg pour 100 ml, n'était que peu soluble dans le mélange alcoolique à 70 % ; par contre, le jaune de quinoléine est soluble mais reste peu visible sur la peau à une concentration de 50 mg pour 100 ml.

Il est apparu que si le jaune de quinoléine est satisfaisant, il n'est pas assez visible sur la peau et qu'il fallait envisager l'ajout d'un second colorant pour renforcer la coloration. Donc, dans un deuxième temps, on ajoute un colorant bleu dans la solution jaune afin d'obtenir une coloration verte ou bleue. Il faut bien entendu respecter les conditions mentionnées ci-dessus et définir la quantité.

Trois colorants additionnels ont été testés avec le jaune de quinoléine : le bleu patenté, l'indigotine et le vert de méthyle. Ce dernier est aussi un indicateur de pH ; il réagit à l'acidité ou l'alcalinité de la solution et, probablement, avec celles de la peau de différents sujets. Il a donc été abandonné. L'indigotine donnait, comme le bleu patenté, une coloration verte satisfaisante, mais comme cela sera décrit dans la suite après conservation l'indigotine est dénaturée. Donc le choix définitif du bleu patenté a été fait et le mélange proposé de produits colorants correspond à la proportion:

| | |
|---|---|
| Jaune de quinoléine | 50 mg par 100 ml, |
| Bleu patenté | 10 mg par 100 ml. |

On va maintenant décrire la fabrication de la solution, au cours de laquelle on procède aux différentes opérations suivantes :
Mélange des produits colorants,
Solubilisation dans l'eau déminéralisée,
Ajout de l'éthanol à 95°,
Ajout du digluconate de chlorhexidine.

La méthode de fabrication d'une unité de solution de 500 g est la suivante :
1) préparation des colorants: on mélange 142 ml d'eau déminéralisée, 250 mg de jaune de quinoléine et 50 mg de bleu patenté ; on agite le mélange pendant 10 minutes ;
2) on ajoute au mélange 422 ml d'alcool éthylique à 95 % et 15 g de chlorhexidine ; on agite l'ensemble pendant 10 minutes ;
3) on procède à une filtration clarifiante sur un filtre 0,22 pm.

Plus en détail, la préparation industrielle de la solution suivant l'invention comporte les étapes suivantes :
*Etape 1 : préparation de la solution colorée :*
   48 heures avant l'étape 3 de fabrication,
   - introduire la totalité de l'eau purifiée, préalablement chauffée à 60° C,
   - mettre l'agitateur en marche à 1500 tr/min,
   - introduire le jaune de quinoléine, en pluie sous agitation continue,
   - laisser agiter pendant 15 minutes,
   - contrôler visuellement l'absence de toute particule colorée,
   - introduire le bleu patenté, en pluie sous agitation continue,
   - laisser agiter pendant 4 heures,
   - laisser reposer pendant 24 heures,
*Etape 2 : filtration de la solution de colorants :*
   - filtrer la solution à l'aide d'un filtre 0,22 µm (carter 20 pouces - Filtre PALL, réf. AB05DFL2PH4 Fluorodine),
   - contrôler la température de la solution qui doit être comprise entre 15° C et 25° C,
   - contrôler visuellement la parfaite dissolution par l'absence de toute particule colorée,
*Etape 3 : achèvement de la fabrication de la solution :*
   - l'alcool à 95 % servant à la fabrication doit avoir été stocké en zone de fabrication, à une température comprise en 15 et 25° C, 3 jours à l'avance,
   - contrôler la température de l'alcool à 95 %, qui doit être comprise entre 15 et 25° C,
   - contrôler la température de la solution de colorants, qui doit être identique à celle de l'alcool à 95 % ou se trouver entre les mêmes limites que précédemment, et vérifier l'absence de particules dans cette solution,
   - mettre l'agitateur en marche à 1500 tr/min,
   - introduire la solution de colorants en 3 fois, avec, à chaque fois, une agitation de 10 minutes,
   - laisser agiter pendant 30 minutes,
   - ajouter lentement le digluconate de chlorhexidine,
   - laisser agiter pendant 2 heures,
*Etape 4 : Repos*
   - laisser reposer, pendant 24 heures, la solution obtenue à la fin de l'étape 3,
   - effectuer un prélèvement pour un contrôle en laboratoire,
*Etape 5 : Filtration et remplissage*
   - filtrer la solution à l'aide d'un filtre 0,22 µm (carter 20 pouces - filtre Pall, réf ; ABO5DFL2PH4 Fluorodine,
   - remplir des flacons de 125 ml, 250 ml ou 500 ml, à l'aide d'une remplisseuse automatique, et les étiqueter,
   - Imprimer, au fond de chaque flacon, la date de péremption et le numéro du lot.

Après la filtration de l'étape 2, la solution obtenue est limpide et de couleur vert émeraude. Au cours de cette filtration, le filtre retient des colorants, ce qui augmente le temps de filtration, et la masse de colorants retenus est de 3,3 mg pour environ 580 ml de solution fabriquée et filtrée.

Dans une variante, en partant de pesées de colorants diminuées de 20 %, après l'ajout ci-dessus d'alcool éthylique, on a procédé à une agitation de 10 minutes avec un chauffage à 40° C, suivi d'un refroidissement; on n'a pas obtenu d'amélioration significative mais il existe un risque de dégrader le principe actif. Cette variante de préparation n'a donc pas été retenue.

On a procédé par la suite à une étude de la stabilité de la solution retenue, en comparant les résultats avec celle de la solution à l'indigotine préparée dans les mêmes conditions que celles mentionnées ci-dessus.

Les solutions à étudier sont versées dans des flacons en polyéthylène de 125, 250 ou 500 ml. Les conditions de conservation sont, pour un flacon, stabilité à 25° C et 65 % H.R. (humidité relative), pour un deuxième, stabilité à 40° C et 75 % H.R. et pour un troisième, stabilité sous U.V. à 365 mm et 30° C. Les études réalisées consistent en le dosage de la chlorhexidine et l'analyse des colorants par une méthode spectrophotométrique.

On a observé que la solution colorée au jaune de quinoléine et bleu patenté a subi une perte d'environ 22 % en jaune de quinolèine et d'environ 10 % en bleu patenté, lors de la filtration clarifiante. Toutefois, cette perte n'engendre aucune conséquence visible sur la coloration vert émeraude, ni sur le pouvoir de coloration de la peau. Après mise en stabilité, les concentrations des colorants n'ont pas sensiblement évolué au cours du temps, quelles que soient les conditions de conservation.

En comparaison, la solution colorée au jaune de quinoléine et indigotine a subi une perte d'environ 22 % en jaune de quinoléine et n'a subi aucune perte pour l'indigotine, lors de la filtration. Par contre, mis en stabilité, la concentration en indigotine a diminué, après 35 jours d'exposition, d'environ 83 % au cours de la conservation sous U.V. à 365 nm et 30° C et d'environ 10 % au cours de la conservation à 25° C et 65 % H.R. La solution placée sous U.V. est de couleur jaune citron. Ces résultats ont conduit à ne pas retenir l'indigotine comme produit colorant à ajouter au jaune de quinoléine.

Reprenant la solution retenue avec, comme colorants, le jaune de quinoléine et le bleu patenté, on a trouvé qu'au bout de 60 jours, les taux de chlorhexidine, dans des conditions de conservation de 25° C à 65% H.R. et 40° C à 75 % H.R., sont compris dans un intervalle de ± 5 % de la valeur théorique. Dans des conditions très sévères, qui ne correspondent pas à la réalité, soit par exemple sous U.V. à 365 nm et 30° C, le taux de chlorhexidine diminue à partir de 30 jours pour atteindre environ 88,8 % de la théorie à 60 jours et montrer, en parallèle, l'apparition du pic de chloroaniline, ce qui révèle une certaine dégradation de la chlorhexidine.

En pratique, après avoir effectué plusieurs essais, la fabrication de la solution suivant l'invention a été optimisée, en respectant les données indiquées dans les étapes 1 à 5, mentionnées ci-dessus. En particulier, la limpidité de la solution a été surveillée tout au long des étapes de fabrication. Cette optimisation de fabrication avait été motivée par l'observation de particules de colorant sur les lots antérieurs après 3 mois de conservation. Ces particules ou grains correspondent à une cristallisation partielle des produits colorants : jaune de quinoléine et bleu patenté.

Il a été montré, lors des études de stabilité de la solution suivant l'invention que cette cristallisation ne concernait que les colorants, qu'elle n'engendrait pas de perte et de dégradation des principes (digluconate de chlorhexidine et alcool), et que le pouvoir colorant sur la peau n'était pas affecté.

La fabrication ayant été optimisée, il a été trouvé que les grains n'apparaissaient qu'à partir de 4 mois, que la taille des grains était très inférieure à 1 mm et qu'ils se localisaient dans la partie supérieure du flacon. L'optimisation du procédé de fabrication a donc permis de retarder le phénomène de cristallisation et de diminuer la taille des grains qui conduisent éventuellement à l'existence d'un léger dépôt.

En résumé, la solution suivant l'invention est une solution prête à l'emploi qui est destinée au badigeonnage du champ opératoire, car elle possède les qualités antiseptiques quand elle est appliquée sur la peau. On doit la laisser sécher avant utilisation d'un instrument électrique, pour éviter toute brûlure. Après l'opération, on l'élimine facilement par simple rinçage à l'eau, ce qui présente une amélioration sensible par rapport à l'utilisation de bétadine.

## Revendications

1. Solution destinée à une application cutanée et, plus particulièrement, à l'antisepsie du champ opératoire qui est préparé en vue d'une intervention chirurgicale, caractérisée en ce qu'elle comprend un principe actif, composé de digluconate de chlorhexidine et d'éthanol, et un produit colorant, composé d'un mélange de deux produits: jaune de quinoléine et bleu patenté, ce qui donne à la solution appliquée sur la peau une coloration verte.

2. Solution suivant la revendication 1, caractérisée en ce qu'elle comprend les constituants suivants pour 100 ml:
solution de digluconate de chlorhexidine à 20 % : 2,66 g
(ce qui correspond à 0,5 g de digluconate de chlorhexidine)
éthanol à 95 % : 75,0 ml
jaune de quinoléine : 44,4 mg
bleu patenté : 8,9 mg
eau purifiée : 25,2 ml

3. Méthode de fabrication de la solution suivant la revendication 2 caractérisée en ce qu'elle comprend les étapes successives suivantes :
- on commence par préparer les colorants, c'est-à-dire qu'on mélange 142 ml d'eau déminéralisée, 250 mg de jaune de quinoléine et 50 mg de bleu patenté, puis on agite le mélange pendant 10 minutes ;
- on ajoute au mélange 422 ml d'alcool éthylique à 95 % et 15 g de chlorhexidine ; on agite l'ensemble pendant 10 minutes ;
- on procède à une filtration clarifiante.

4. Méthode de fabrication de la solution suivant la revendication 2, caractérisée en ce qu'elle comprend les étapes successives suivantes :
*Etape 1 : préparation de la solution colorée :*
48 heures avant l'étape 3 de fabrication,
- introduire la totalité de l'eau purifiée, préalablement chauffée à 60° C,
- mettre l'agitateur en marche à 1500 tr/min,
- introduire le jaune de quinoléine, en pluie sous agitation continue,
- laisser agiter pendant 15 minutes,
- contrôler visuellement l'absence de toute particule colorée,
- introduire le bleu patenté, en pluie sous agitation continue,
- laisser agiter pendant 4 heures,
- laisser reposer pendant 24 heures,
*Etape 2 : filtration de la solution de colorants :*
- filtrer la solution à l'aide d'un filtre 0,22 µm,
- contrôler la température de la solution qui doit être comprise entre 15° C et 25° C,
- contrôler visuellement la parfaite dissolution par l'absence de toute particule colorée,
*Etape 3 : achèvement de la fabrication de la solution :*
- l'alcool à 95 % servant à la fabrication doit avoir été stocké en zone de fabrication, à une température comprise en 15 et 25° C, 3 jours à l'avance,
- contrôler la température de l'alcool à 95 %, qui doit être comprise entre 15 et 25° C,
- contrôler la température de la solution de colorants, qui doit être identique à celle de l'alcool à 95 % ou se trouver entre les mêmes limites que précédemment, et vérifier l'absence de particules dans cette solution,
- mettre l'agitateur en marche à 1500 tr/min,
- introduire la solution de colorants en 3 fois, avec, à chaque fois, une agitation de 10 minutes,
- laisser agiter pendant 30 minutes,
- ajouter lentement le digluconate de chlorhexidine,
- laisser agiter pendant 2 heures,
*Etape 4 : Repos*
- laisser reposer, pendant 24 heures, la solution obtenue à la fin de l'étape 3,
- effectuer un prélèvement pour un contrôle en laboratoire,
*Etape 5 : Filtration et remplissage*
- filtrer la solution à l'aide d'un filtre 0,22 µm (carter 20 pouces - filtre Pall, réf ; ABO5DFL2PH4 Fluorodine,
- remplir des flacons de 125 ml, 250 ml ou 500 ml, à l'aide d'une remplisseuse automatique, et les étiqueter,
- Imprimer, au fond de chaque flacon, la date de péremption et le numéro du lot.

5. Moyen de fabrication destiné à mettre en oeuvre la méthode de la revendication 3, caractérisé en ce que, pour la filtration clarifiante, il est utilisé un filtre 0,22 µm.

6. Solution suivant une des revendications 1 ou 2, ou fabriquée suivant une des méthodes 3 ou 4, caractérisée en ce qu'elle est conservée dans des flacons opaques en polyéthylène haute densité.
